(19) Europäisches Patentamt
European Patent Office
Office européen des brevets

(11) Publication number: **0 394 868 A1**

(12) **EUROPEAN PATENT APPLICATION**

(21) Application number: **90107530.9**

(22) Date of filing: **20.04.90**

(51) Int. Cl.[5]: **C07C 291/10, A61K 31/275, A01N 47/40, C12P 13/00**

(30) Priority: **26.04.89 JP 104713/89**
**12.10.89 JP 263870/89**

(43) Date of publication of application:
**31.10.90 Bulletin 90/44**

(84) Designated Contracting States:
**DE FR GB IT**

(71) Applicant: **Nippon Kayaku Kabushiki Kaisha**
**11-2, Fujimi-cho 1 chome Chiyoda-ku**
**Tokyo(JP)**

(72) Inventor: **Nishimoto, Masakazu**
**1039 Kamiochiai**
**Yono-shi, Saitama-ken(JP)**
Inventor: **Morino, Tomio**
**1090 Kamiochiai**
**Yono-shi, Saitama-ken(JP)**
Inventor: **Takada, Kimihiko**
**1-5-16, Umesato, Suginami-ku**
**Tokyo(JP)**
Inventor: **Sato, Masaya**
**6-3-12 Megurohon-cho, Meguro-ku**
**Tokyo(JP)**
Inventor: **Takagi, Kenkichi**
**8-22-7 Oizumigakuen-cho, Nerima-ku**
**Tokyo(JP)**
Inventor: **Seya, Kenji**
**4-21-3 Minami-cho**
**Warabi-shi, Saitama-ken(JP)**
Inventor: **Nishikiori, Takaaki**
**2-12-2-201 Wakabayashi, Setagaya-ku**
**Tokyo(JP)**

(74) Representative: **Türk, Gille, Hrabal**
**Brucknerstrasse 20**
**D-4000 Düsseldorf 13(DE)**

(54) **New antibiotics NK372135, process for the production thereof and use thereof.**

(57) The present invention relates to antibiotics NK372135, which have antibacterial and antifungal actions and a 5-lipoxygenase inhibitory activity, represented by the following formula:

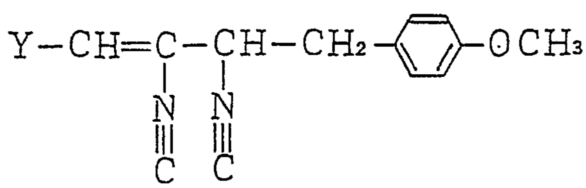

(I)

wherein Y represents a 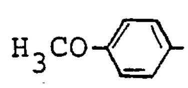, 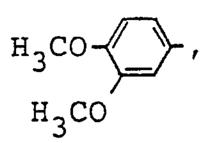,

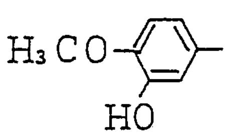 or 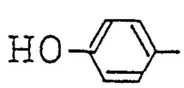 group,

or salts thereof and a process for the production of the same by fermentation.

EP 0 394 868 A1

Fig. 1

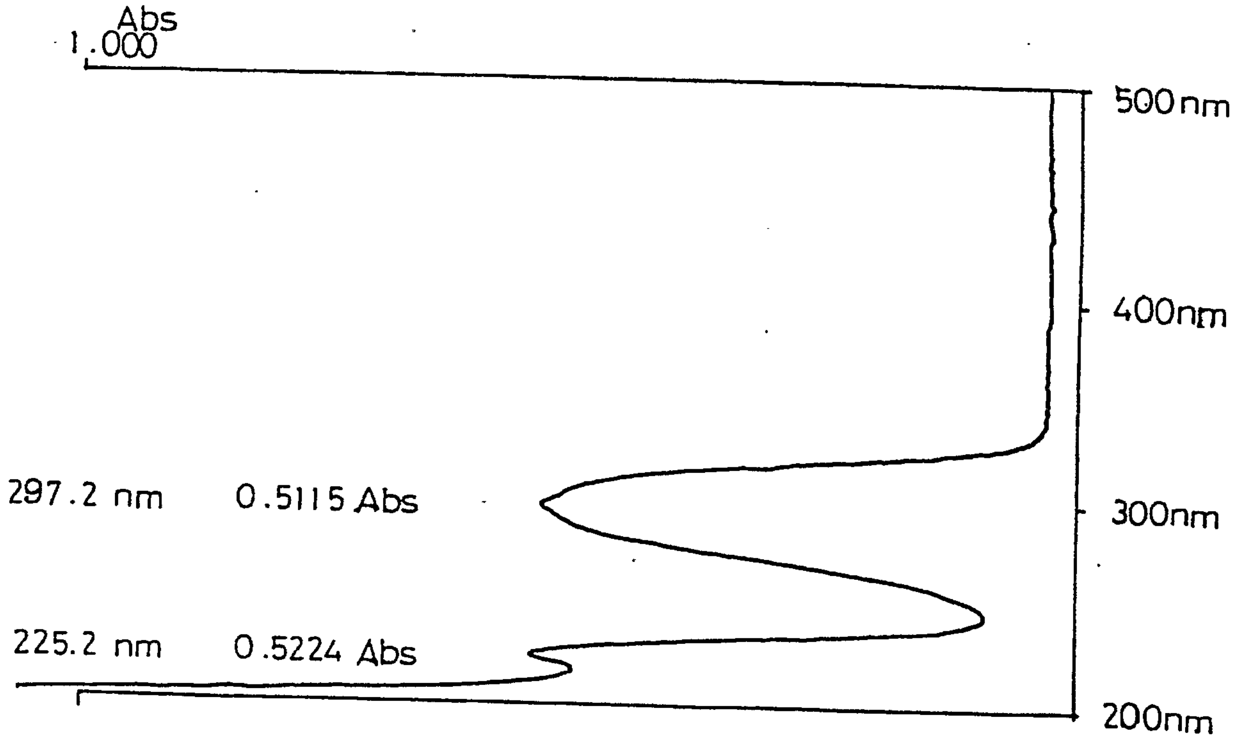

Abs
1.000
500nm
400nm
300nm
200nm
297.2 nm
0.5115 Abs
225.2 nm
0.5224 Abs

# NEW ANTIBIOTICS NK372135, PROCESS FOR THE PRODUCTION THEREOF AND USE THEREOF

## BACKGROUND OF THE INVENTION

Known antifungal agents include amphotericin B, fluorocytosine and miconazole.

However none of these known antifungal agents is satisfactory from the viewpoints of, for example, toxicity and potency. Therefore it has been urgently required to find a novel compound suitable for these uses.

## SUMMARY OF THE INVENTION

Under these circumstances, the present inventors have examined various metabolites of microorganisms. As a result, they have found out that a mold strain belonging to the genus Neosartoria produces antibiotics NK372135A, B, C and D having an antifungal action, a 5-lipoxygenase inhibitory activity and other activities.

Each of the novel antibiotics NK372135A, B, C and D according to the present invention has antibacterial and antifungal actions and a 5-lipoxygenase inhibitory activity. Thus they are expected to be useful as a chemotherapeutic agent for bacterial and fungal infections, as a remedy for allergic diseases, asthma and ischemic heart diseases and as an agricultural and horticultural antifungal agent.

## DETAILED DESCRIPTION OF THE INVENTION

The present invention has been completed based on the above-mentioned finding.

The antibiotics of the present invention are represented by the following formula:

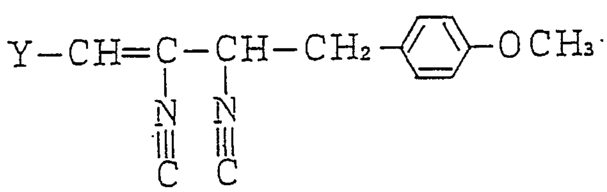

wherein Y represents a 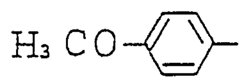, 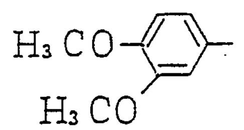,

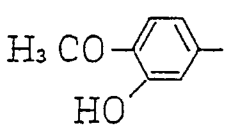 or 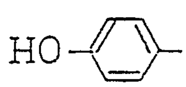 group.

The compounds of the present invention have the following physicochemical properties.

(a) Antibiotic NK372135A

1) Appearance: yellow powder.
2) Molecular weight: FD-MS m/z 318 ($M^+$).

3) Rational formula: $C_{20}H_{18}O_2N_2$.

4) Solubility: soluble in lower alcohols, chloroform and acetates.

5) Rf in silica gel thin-layer chromatography (Merck Art. 5715): 0.66 and 0.23 with, respectively, chloroform/methanol (100 : 2) and hexane/acetone (3 : 1) each as a developing solvent.

6) UV absorption spectrum: as shown in Fig. 1.

7) IR absorption spectrum: as shown in Fig. 2 (according to potassium bromide tablet method).

8) Hydrogen nuclear magnetic resonance spectrum:
as shown in Fig. 3 (in heavy methanol).

9) Carbon nuclear magnetic resonance spectrum:
as shown in Fig. 4 (in heavy methanol).

10) Color change test: positive to phosphomolybdic acid reaction, sulfuric acid and iodine, and negative to ninhydrin reaction.

(b) Antibiotic NK372135B

1) Appearance: yellow powder.

2) Molecular weight: FD-MS m/z 348 ($M^+$).

3) Rational formula: $C_{21}H_{20}O_3N_2$.

4) Solubility: soluble in lower alcohols, chloroform and acetates.

5) Rf in silica gel thin-layer chromatography (Merck Art. 5715): 0.40 and 0.16 with, respectively, chloroform/methanol (100 : 2) and hexane/acetone (3 : 1) each as a developing solvent.

6) UV absorption spectrum: as shown in Fig. 5.

7) IR absorption spectrum: as shown in Fig. 6 (according to potassium bromide tablet method).

8) Hydrogen nuclear magnetic resonance spectrum:
as shown in Fig. 7 (in heavy chloroform).

9) Carbon nuclear magnetic resonance spectrum:
as shown in Fig. 8 (in heavy chloroform).

10) Color change test: positive to phosphomolybdic acid reaction, sulfuric acid and iodine, and negative to ninhydrin reaction.

(c) Antibiotic NK372135C

1) Appearance: yellow powder.

2) Molecular weight: FD-MS m/z 334 ($M^+$).

3) Rational formula: $C_{20}H_{18}O_3N_2$.

4) Solubility: soluble in lower alcohols, chloroform and acetates.

5) Rf in silica gel thin-layer chromatography (Merck Art. 5715): 0.13 and 0.14 with, respectively, chloroform/methanol (100 : 2) and hexane/acetone (3 : 1) each as a developing solvent.

6) UV absorption spectrum: as shown in Fig. 9.

7) IR absorption spectrum: as shown in Fig. 10 (according to potassium bromide tablet method).

8) Hydrogen nuclear magnetic resonance spectrum:
as shown in Fig. 11 (in heavy chloroform).

9) Carbon nuclear magnetic resonance spectrum:
as shown in Fig. 12 (in heavy chloroform).

10) Color change test: positive to phosphomolybdic acid reaction, sulfuric acid and iodine, and negative to ninhydrin reaction.

(d) Antibiotic NK372135D

1) Appearance: yellow powder.

2) Molecular weight: FD-MS m/z 304 ($M^+$).

3) Rational formula: $C_{19}H_{16}O_2N_2$.

4) Solubility: soluble in lower alcohols, chloroform and acetates.

5) Rf in silica gel thin-layer chromatography (Merck Art. 5715): 0.56 and 0.14 with, respectively, chloroform/methanol (100 : 2) and hexane/acetone (3 : 1) each as a developing solvent.

6) UV absorption spectrum: as shown in Fig. 13.

7) IR absorption spectrum: as shown in Fig. 14 (according to potassium bromide tablet method).

8) Hydrogen nuclear magnetic resonance spectrum:

as shown in Fig. 15 (in heavy chloroform).

9) Carbon nuclear magnetic resonance spectrum:

as shown in Fig. 16 (in heavy chloroform).

10) Color change test: positive to phosphomolybdic acid reaction, sulfuric acid and iodine, and negative to ninhydrin reaction.

Now the structural formulae of NK372135A, NK372135B, NK372135C and NK372135D will be given.

NK372135A:

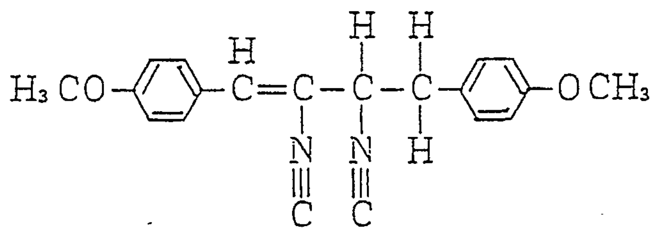

NK372135B:

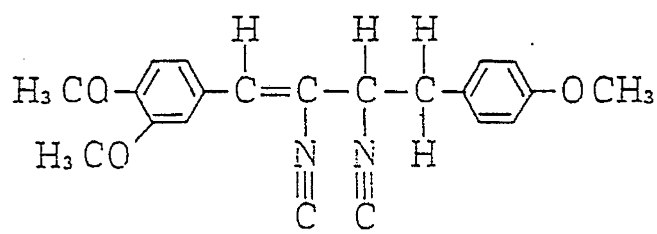

NK372135C:

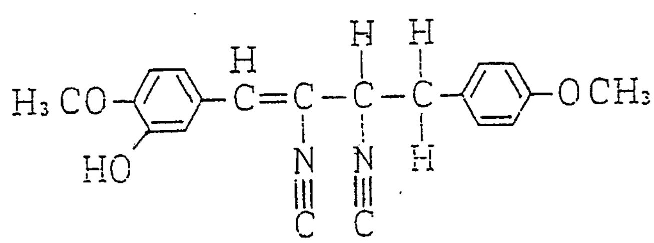

NK372135D:

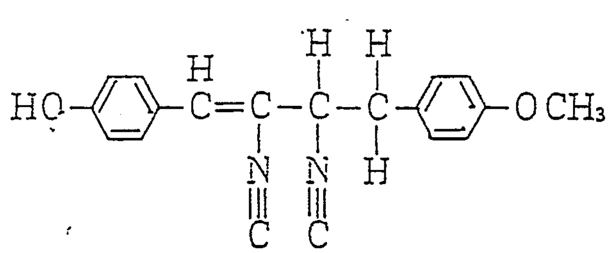

As an example of the strain belonging to the genus Neosartoria which can produce the compound of

the present invention, namely, NK372135A, B, C or D, Neosartoria fischeri var. glabra [IFO 9857, Tubaki, K. et al., Ann. Micr., 24, 199 (1974)] may be cited.

The Neosartoria fischeri var. glabra strain to be used in the present invention may be subjected to commonly employed breeding treatments (for example, irradiation with UV light or $^{60}Co$, mutagenesis with the use of a mutagenic agent such as nitrogen mustard, nitrous acid, N-methyl-N′-nitro-nitrosoguanidine (NTG) or 2-aminopurine, transduction, transformation or cell fusion) so as to enhance the productivity of the NK372135A, B, C or D.

The NK372135A, B, C or D of the present invention may be produced by culturing a microorganism, which belongs to the genus Neosartoria and is capable of producing NK372135A, B, C or D, in a nutritional medium and collecting the NK372135A, B, C or/and D thus accumulated in the culture medium.

The culture can be conducted in accordance with a method commonly employed for culturing a mold. It is generally advantageous to conduct the culture by the submerged method. Any medium may be used therefor so long as it contains nutritional sources utilized by the Neosartoria fischeri var. glabra strain.

As the nutritional sources, known materials heretofore utilized in the culture of molds may be used. Examples of the carbon source include glucose, galactose, sucrose, lactose, glycerol, dextrin, starch, starch syrup (saccharified starch malt) and soybean oil. Either one of these materials of a mixture thereof may be used. Examples of the inorganic and organic nitrogen sources include ammonium chloride, ammonium sulfate, urea, ammonium nitrage, sodium nitrate, peptone, meat extract, yeast extract, dry yeast, corn steep liquor, soybean flour, pharma media, oatmeal, casamino acids, bacto-soytone and soluble vegetable proteins. Either one of these materials or a mixture thereof may be used. Furthermore, the medium may contain various additives, for example, inorganic salts such as common salt, magnesium sulfate, copper sulfate, zinc sulfate, manganese chloride, calcium carbonate and phosphates and organic and inorganic materials promoting the growth of the mold or enhanging the production of the antibiotics NK372135A, B, C or D, for example, nucleic acids, amino acids, vitamins or inorganic matters, if required. When vigorous foaming is observed during the culture, a vegetable oil such as soybean oil or linseed oil or a petroleum defoamer such as Pronal 1 (a product of Toho Chemical Industry Co., Ltd.) or Silicone KM-70 (a product of The Shin-Etsu Chemical Co., Ltd.) may be added. The culture may be preferably conducted at a temperature of from about 20 to about 30° C under neutral or slightly acidic conditions. In the case of the liquid culture, NK372135A, B, C or/and D may be accumulated in the medium generally within three to eight days. When the maximum accumulation of the metabolite(s) in the medium is achieved, the culture is ceased and the cells are recovered by filtration. The target product(s) may be isolated and purified from the cells and the filtrate.

The isolation and purification of the product(s) from the culture medium may be carried out by a method commonly employed for isolating and purifying a microbial metabolite from the culture medium. Since NK372135A, B, C and D are soluble in organic solvents such as methanol, acetone, ethyl acetate and ethanol but hardly soluble in water, a method commonly employed for the purification of a fat-soluble material may be employed therefor.

Namely, extraction with various organic solvents, silica gel chromatography, gel filtration and a combination of these procedures may be appropriately used therefor.

For example, the culture medium is divided into the cells and the filtrate by filtering. Next, the cells are extracted with methanol twice and the methanol extracts are concentrated to dryness under reduced pressure. On the other hand, the filtrate is extracted with butanol and the butanol extract is concentrated to dryness under reduced pressure. Each brown crude powder thus obtained is then dissolved in chloroform/methanol (1 : 1) and purified by silica gel chromatography. The development is conducted stepwise (100 : 0, 100 : 2 and 2 : 1) by using chloroform/methanol as a solvent. Thus active fractions I, II and III are obtained. Each active fractions are combined and concentrated to dryness to thereby give a brown powder.

The powder obtained from each active fraction is then dissolved in chloroform/methanol (1 : 1) and extracted and purified by silica gel chromatography (scratching). Hexane/acetone (3 : 1) is used as the developing solvent while chloroform/methanol (1 : 1) is used for extracting. Thus NK372135A, NK372135B and NK372135C and NK372135D are obtained, respectively, from the active fractions I, II and III each in the form of a yellow powder.

Each powder is then dissolved in methanol and purified by LH-20 chromatography to thereby give a yellow powder.

The titers of NK372135A, B, C and D in the culture medium and crude fraction are determined by measuring the growth inhibitory effects on Candida albicans strain cultured in an agar plate medium.

As will be described hereinafter, the invention compounds NK372135A, B, C and D are expected to be available as an antibacterial or antifungal agent, a drug for treating allergic diseases, astham or ischemic

heart diseases or as an agricultural and horticultural antifungal agent.

When the compound of the present invention is to be used as a drug, it may be formulated into, for example, injection, oral preparation or suppository either alone or together with fillers. Any filler may be used so long as it is pharmaceutically acceptable. Thus the fillers and the compositions thereof may be appropriately selected depending on the administration pathway and administration manner. For example, liquid fillers may be selected from among water, alcohols and animal, vegetable and synthetic oils such as soybean oil, peanut oil, sesame oil and mineral oils. Further, solid fillers may be selected from among sugars such as maltose and sucrose, various amino acids, cellulose derivatives such as hydroxypropylcellulose and organic acid salts such as magnesium stearate.

When the compound of the present invention is to be formulated into an injection, preferable examples of the fillers include physiological saline solution, various buffer solutions, solutions of sugars such as glucose, inositol and mannitol and solutions of glycols such as ethylene glycol and polyethylene glycol. Alternately, the compound of the present invention may be lyophilized together with fillers, for example, sugars such as inositol, mannitol, glucose, mannose, maltose or sucrose or amino acids such as phenylalanine, and the obtained lyophilized preparation is dissolved in an appropriate solvent for injection such as sterilized water, a glucose solution, an electrolyte solution or an amino acid solution and intravenously or intramuscularly injected.

In the case of an oral preparation, the compound of the present invention may be formulated into tablet, capsule, dust, granules, syrup or dry syrup together with the above-mentioned liquid or solid fillers. Furthermore, it may be formulated into pellets for topical application such as percutaneous or mucosal application.

The content of the compound of the present invention in a preparation generally ranges from about 0.001 to about 1% by weight, preferably from about 0.01 to about 0.1% by weight. For example, an injection preferably contains the compound of the present invention in an amount of about 0.01 to about 0.05% by weight in general. On the other hand, an oral preparation may contain the compound of the present invention in an amount of from about 0.005 to about 1% by weight, preferably from about 0.05 to about 0.5% by weight, while the balance comprises fillers.

The dose of the compound of the present invention is determined depending on the age, body weight and conditions of the patient and the purpose of the treatment. It generally ranges from about 0.1 to about 5 µg/kg/day in the case of parenteral administration and from about 0.5 to about 30 µg/kg/day in the case of oral administration.

When the compound of the present invention is to be used as an effective remedy for allergic diseases, asthma or ischemic heart diseases, it may be either orally or parenterally administered. The dose may preferably ranges from about 0.01 to about 20 µg/kg/day in general, though it varies depending on the administration manner.

The compound of the present invention is administered in the form of a preparation obtained by mixing the same with appropriate pharmaceutical carriers. Namely, it may be formulated into, for example, tablet, granules, fine granules, dust, capsule, suppository, injection, ointment or aerosol.

When the compound of the present invention is to be used as an agricultural and horticultural antifungal agent, it may be used as such depending on the purpose. However it may be generally blended with agricultural auxiliary substances for further enhancing the effect and/or stabilizing the compound. The preparation thus obtained may be used either as such or after dilution, if desired. The formulation of the compound of the present invention is not particularly restricted. Thus it may be formulated into arbitrary forms, for example, dust, granules, fine granules, wettable powder, flowable, emulsion, microcapsule, oil solution, aerosol, thermal fumigant such as mosquito-repellent incense or electric mosquito-repellent, hazing agent such as fogging, nonthermal fumigant and poisonous feed by a method commonly employed in the art.

The term "agricultural auxiliary substance" as used herein involves carriers (diluents) and other auxiliary substances such as spreaders, emulsifiers, dispersing agents, sticking agents and disintegrating agents.

The dosage may vary depending on, for example, the form, application method and application time. Generally speaking, an agricultural or horticultural chemical may be used in such an amount as to give about 10 to about 300 g, preferably about 15 to about 200 g, of the active ingreident(s) per 10 a.

(Function)

The activity and toxicity of NK372135A, B, C and D are described as follows:

Test Example 1

The antimicrobial spectra of the compounds of the present invention were examined.

Table 1 gives the antimicrobial spectra of NK372135A, C and D. A paper disc of 8 mm in diameter was immersed in a solution containing 100 µg/ml of NK372135A, C or D and then placed on an agar medium in which a test strain had been grown. Regarding the four strains listed in the upper part of Table 1 and Candida albicans, each culture was conducted at 37 °C for a day and night, while other strains were cultured at 25 °C for a day and night. Then the diameter of each inhibition zone thus formed was measured. It was found that NK372135A, C and D strongly inhibited the growth of bacteria including Escherichia coli and Bacillus subtilis, fungi belonging to the genera Aspergillus, Trichophyton and Candida, which are known as the major pathogenic fungi causing animal and human fungal diseases, and a fungus belonging to the genus Botrytis, which is known as a pathogenic fungus causing gray mold in plants. Since no existing antifungal agent exerts a satisfactory effect on fungi, the compounds of the present invention are highly expected as effective antifungal agents.

Table 1

| | Diameter of inhibition zone (mm) | | |
|---|---|---|---|
| Test strain | NK372135A | NK372135C | NK372135D |
| Escherichia coli | 20 mm | 29 mm | 25.5 mm |
| Bacillus subtilis | 21.5 mm | 28 mm | 26 mm |
| Staphylococcus aureus | 20.5 mm | 26 mm | 28 mm |
| Mycobacterium smegmatis | 21.0 mm | - | - |
| Mucor japonicus | 15 mm | 17 mm | 16 mm |
| Aspergillus oryzae | 20 mm | 21 mm | 20 mm |
| Mycosphaerella melonis | 18 mm | 19 mm | 18 mm |
| Fusarium roseum | 9 mm | 12 mm | 12 mm |
| Botrytis fabae | 25 mm | 25 mm | 25 mm |
| Trichophyton mentagrophytes | 30 mm | 30 mm | 30 mm |
| Saccharomyces cerevisiae | 25 mm | 25 mm | 25 mm |
| Candida albicans | 30 mm | 25 mm | 24 mm |

Test Example 2

The 5-lipoxygenase inhibitory activities of the compounds of the present invention were examined.

Table 2 gives the inhibitory activities of NK372135A, B and C.

The 5-lipoxygenase inhibitory activity was evaluated from the inhibitory activity on ionophore stimulation on Guinea pig peritoneal cells in the following manner.

A 2% casein solution was intraperitoneally administered to Hartley Guinea pigs weighing 250 to 350 g. 18 hours after the administration, the peritoneal cells of the animals were collected. The contaminating erythrocytes were hemolyzed and centrifuged. To the cell fraction thus obtained was added a 50 mM phosphate buffer solution (pH 7.0) containing 14 mM of indomethacin and the concentration of the peritoneal cells was adjusted to 1 x $10^7$ cell/ml. To 1.0 ml of the cell suspension thus obtained, a test compound of various concentrations was added so as to give a final concentration of 100 µM. Then the mixture was incubated at 37 °C for five minutes. Next, 1.0 µg of Ionophore A 23187 and 10 µg of arachidonic acid were added thereto and the reaction was initiated. After 20 minutes, the reaction was ceased by adding 4 ml of ethanol. The reaction supernatant was analyzed by HPLC in accordance with the method of Amano et al. [Vitamin, 59, 211 - 219 (1985)] so as to detect 5-hydroxyeicosatetraenoic acid (5-HETE) which was a metabolite derived from arachidonic acid.

Table 2

| | Inhibition ratio (%) | | |
|---|---|---|---|
| Compound | $10^{-4}$ mol/ℓ | $10^{-5}$ mol/ℓ | $10^{-6}$ mol/ℓ |
| NK372135A | 37.1 | 5.0 | 0 |
| NK372135B | 33.3 | 5.0 | 0 |
| NK372135C | 100 | 25.5 | 12.9 |

Thus it was found out that NK372135A, B and C had 5-lipoxygenase inhibitory activities.

Test Example 3: Anti-Candida activity

The anti-Candida activity of NK372135B was compared with that of methoxy-xanthocillin X dimethyl ether which is shown in The Journal of Antibiotics Vol 34 (1981) No. 12, P1556 ~ 1561.

Candida albicans was inoculated into a 96-well plate at a density of 2 x $10^3$ cell/well with the use of 0.2 cc of a bouillon medium. Then a test compound of various concentrations was added thereto and the microorganism was incubated at 30°C for 16 hours. Then the absorbance of each well at 595 nm was measured and $IC_{50}$ was calculated from the dose response curve. It was found that the $IC_{50}$ of the NK372135B was 1.5 μg/ml while that of the methoxy-xanthocillin X dimethyl ether was 9.0 μg/ml. Thus it was found out that the saturation of the double bond in methoxy-xanthocillin X dimethyl ether enhanced the anti-Candida activity.

Test Example 4: Cytotoxicity

The cytotoxicities of NK372135A, B and C and methoxy-xanthocillin monomethyl ether (XTM) which is an analog thereof were examined. 2,000 cells of each of G361, SW1116, PC-3 and Li-7 cell strains were inoculated into a 96-well plate and incubated at 37°C under 5% $CO_2$ for 24 hours. An RPMI 1640 medium containing 10% of fetal calf serum was employed as a medium. After incubating for 24 hours, a test compound of a given concentration was added thereto and the incubation was further conducted for two to four days. Subsequently, the cells were counted by the methylene blue method so as to evaluate the extent of the growth inhibition. Table 3 gives the results.

Table 3:

| Cytotoxicity | | | | |
|---|---|---|---|---|
| | $IC_{50}$ (μg/ml) | | | |
| Cell strain | A | B | C | XTM* |
| G361 | 2.13 | 2.40 | 2.52 | - |
| SW1116 | 1.65 | 2.55 | 5.89 | - |
| PC-3 | 1.05 | 0.76 | 1.23 | - |
| Li-7 | 1.45 | 1.80 | 1.70 | 0.57 |

Thus it was found that the cytotoxicities of the NK372135 antibiotics were lower than that of XTM.

These results suggest that NK372135A, B, C and D have antibacterial, antifungal and 5-lipoxygenase inhibitory activities and that they are thus expected to be useful as a novel antibacterial and antifungal agent for medical, agricultural, horticultural and livestock uses and as a remedy for allergic diseases, asthma and

ischemic heart diseases.

To illustrate the process for the production of the compound of the present invention, the following Example will be given.

Example 1

100 ml of a medium (pH 6.8) comprising 2% of glucose, 1% of glycerol, 1% of lactose, 1% of sucrose, 3% of soybean flour, 0.8% of polypeptone, 0.2% of sodium nitrage and 0.1% of magnesium sulfate was pipetted into a 500-ml Erlenmeyer flask and sterilized in an autoclave at 120 °C for 20 minutes. Then the medium was inoculated with one platinum loopful of IFO 9857 strain. Then the strain was cultured at 27 °C in a rotary shaker under shaking at 200 rpm for two days. Separately, 100 ml of the above-mentioned medium was pipetted into a 500-ml Erlenmeyer flask and sterilized in an autoclave at 120 °C for 20 minutes. Then it was inoculated with 1 ml of the above-mentioned culture medium, which was then cultured under shaking in a rotary shaker for three days.

Next, 20-1 of the above-mentioned medium was pipetted into a 30-1 jar fermenter and sterilized at 120 °C for 30 minutes. 200 ml of the above-mentioned culture medium was transplanted into this jar fermenter and cultured at 25 °C under aerating at a rate of 20-1/min at 300 rpm.

30-1 of the culture medium was then filtered and the cell fraction was extracted by adding 18-1 of methanol and stirring. On the other hand, the filtrate fraction was extracted by adding 18-1 of butanol and stirring.

The methanol and butanol extracts thus obtained were concentrated under reduced pressure to thereby give 105 g of a brown lyophilized powder.

This powder was dissolved in chloroform/methanol (1 : 1) and poured into 5.0-1 of a silica gel column which had been equilibrated with chloroform. Then it was eluted while varying the ratio of chloroform to methanol stepwise, i.e., 100 : 1, 100 : 2 and 2 : 1.

The active fractions (I), (II) and (III) were each combined and concentrated to dryness under reduced pressure. Thus 1.2 g, 0.3 g and 0.7 g of pale brown powders (I), (II) and (III) were obtained, respectively. These powders were dissolved in chloroform/methanol (1 : 1) again and subjected to silica gel column chromatography with the use of chloroform/methanol (100 : 2) as a developing solvent. Active fractions obtained from each column were combined and concentrated to dryness under reduced pressure. Thus 200 mg of a yellow powder (A) was obtained from the active fraction (I), 50 mg of a yellow powder (B) was obtained from the active fraction (II) and 80 mg of a yellow powder (C) and 30 mg of the yellow powder (D) were obtained from the active fraction (III). Each powder was dissolved in methanol and subjected to gel filtration chromatography with the use of Sephadex LH-20. Active fractions were combined and concentrated to dryness under reduced pressure. Thus 120 mg of NK372135A, 30 mg of NK372135B, 46 mg of NK372135C and 15 mg of NK372135D were obtained.

4. Brief Description of the Drawings:

Figs. 1, 2, 3 and 4 respectively show the UV absorption spectrum, IR absorption spectrum, hydrogen nuclear magnetic resonance spectrum and carbon nuclear magnetic resonance spectrum of the antibiotic NK372135A. Figs. 5, 6, 7 and 8 respectively show the UV absorption spectrum, IR absorption spectrum, hydrogen nuclear magnetic resonance spectrum and carbon nuclear magnetic resonance spectrum of the antibiotic NK372135B. Figs. 9, 10, 11 and 12 respectively show the UV absorption spectrum, IR absorption spectrum, hydrogen nuclear magnetic resonance spectrum and carbon nuclear magnetic resonance spectrum of the antibiotic NK372135C. Figs. 13, 14, 15 and 16 respectively show the UV absorption spectrum, IR absorption spectrum, hydrogen nuclear magnetic resonance spectrum and carbon nuclear magnetic resonance spectrum of the antibiotic NK372135D.

## Claims

1. Antibiotics NK372135 represented by the following formula:

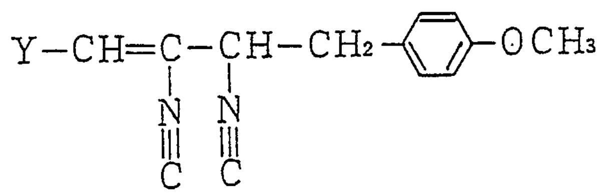 (I)

wherein Y represents a 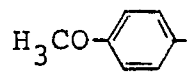, 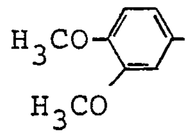,

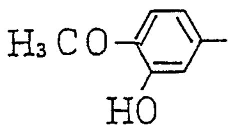 or 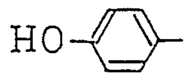 group,

or a salt thereof.

2. A process for the production of antibiotics NK372135 represented by the above formula (I) or salts thereof, which comprises culturing a microorganism, which belongs to the genus Neosartoria and is capable of producing said antibiotics NK372135, in a medium and collecting the antibiotics NK372135 thus accumulated in the medium.

3. An antifungal agent or a 5-lipoxygenase inhibitor comprising at least one of the antibiotics NK372135 represented by the above formula (I) or salts thereof as an active ingredient.

4. A pharmaceutical composition containing compounds of claim 1 as active ingredients in association with the pharmaceutically acceptable, substantially non-toxic carrier or excipient.

5. A compound of claim 1 for use as a medicament.

6. Use of compounds of claim 1 for manufacture of medicament for treating bacterial and fungal infections, allergic diseases, asthma, or ischemic heart diseases.

7. Use of compounds of claim 1 as an agricultural or horticultural antifungal agent.

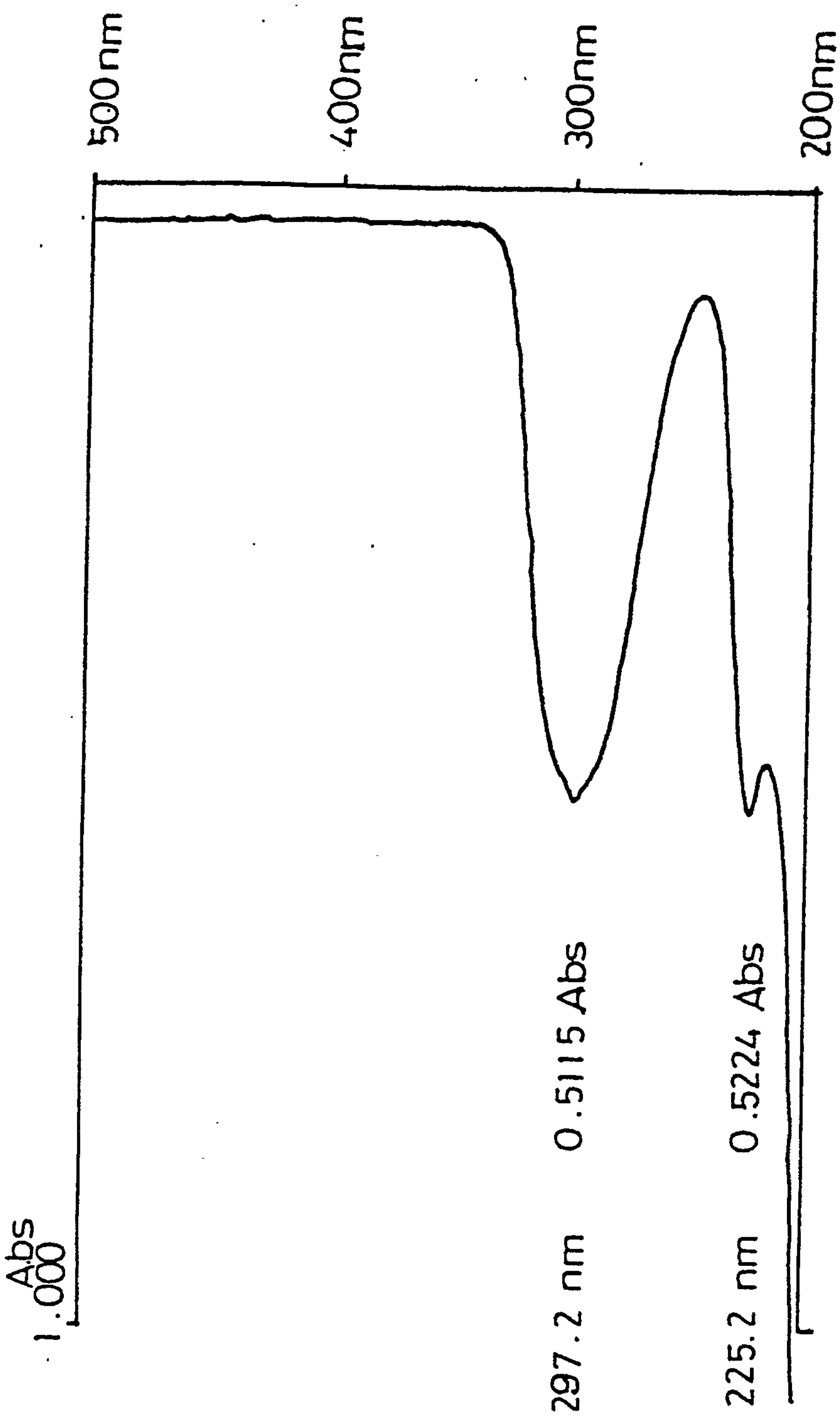
500nm
400nm
300nm
200nm
Abs
1.000
297.2 nm
0.5115 Abs
225.2 nm
0.5224 Abs

Fig. 1

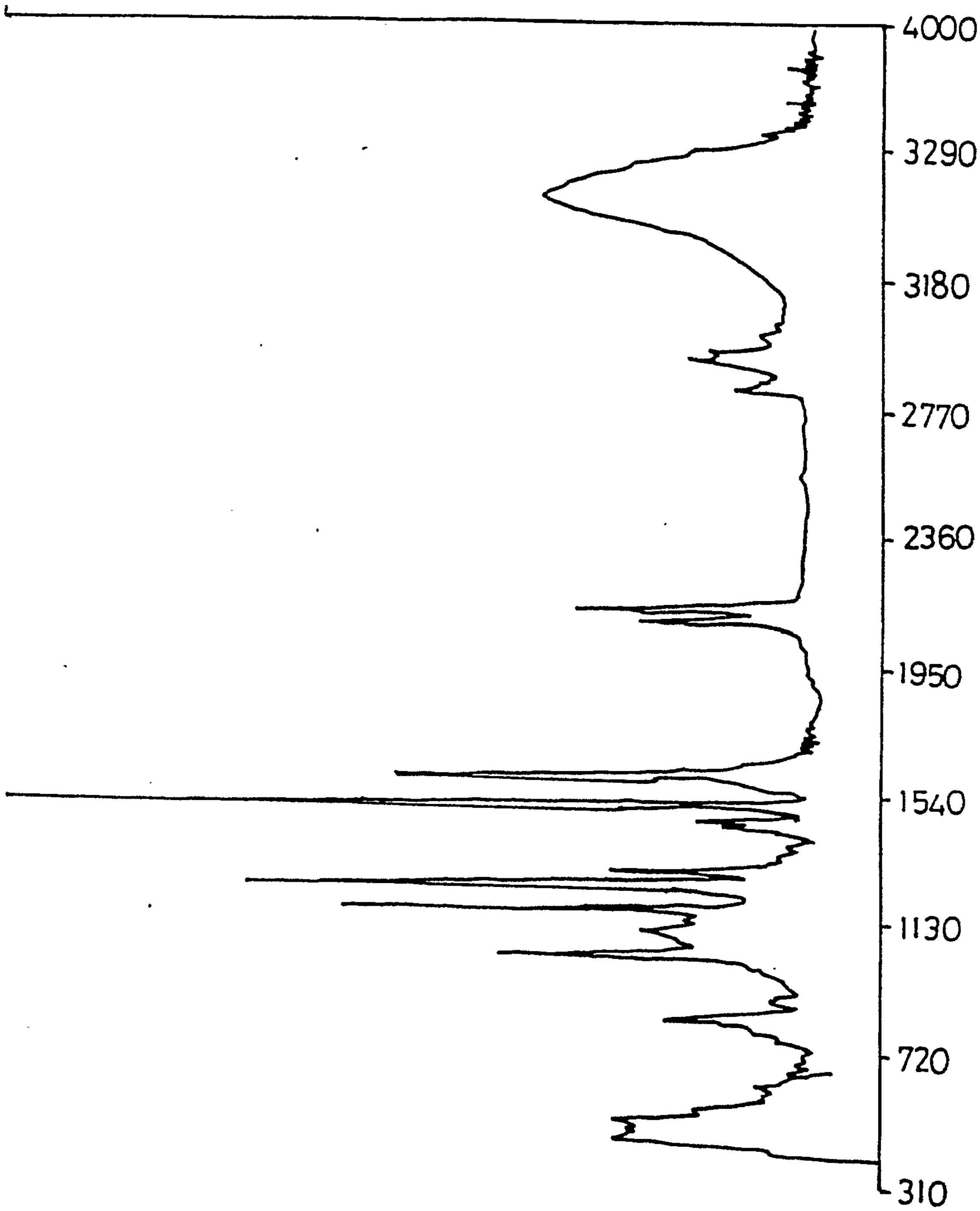


Fig. 2

Fig.3

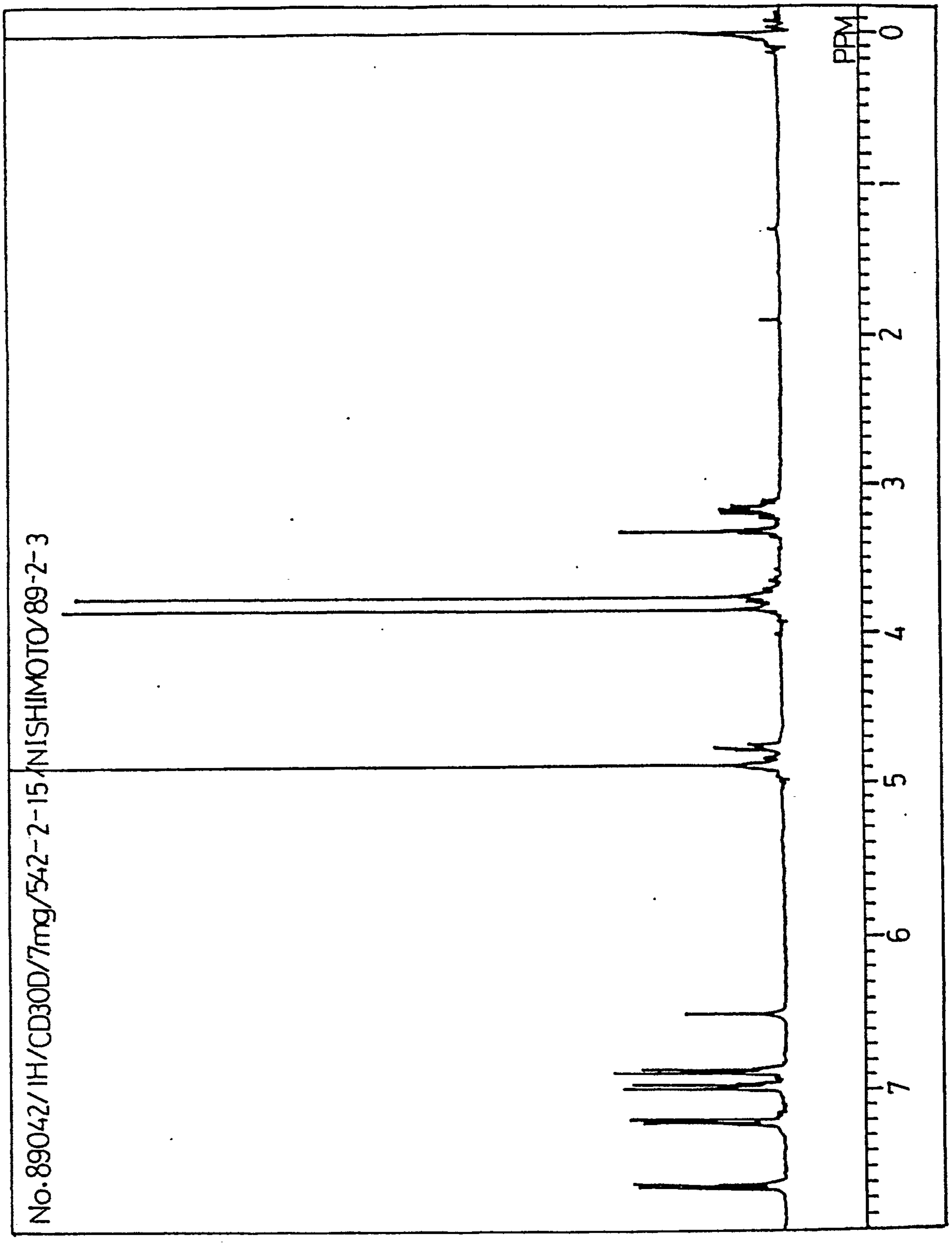

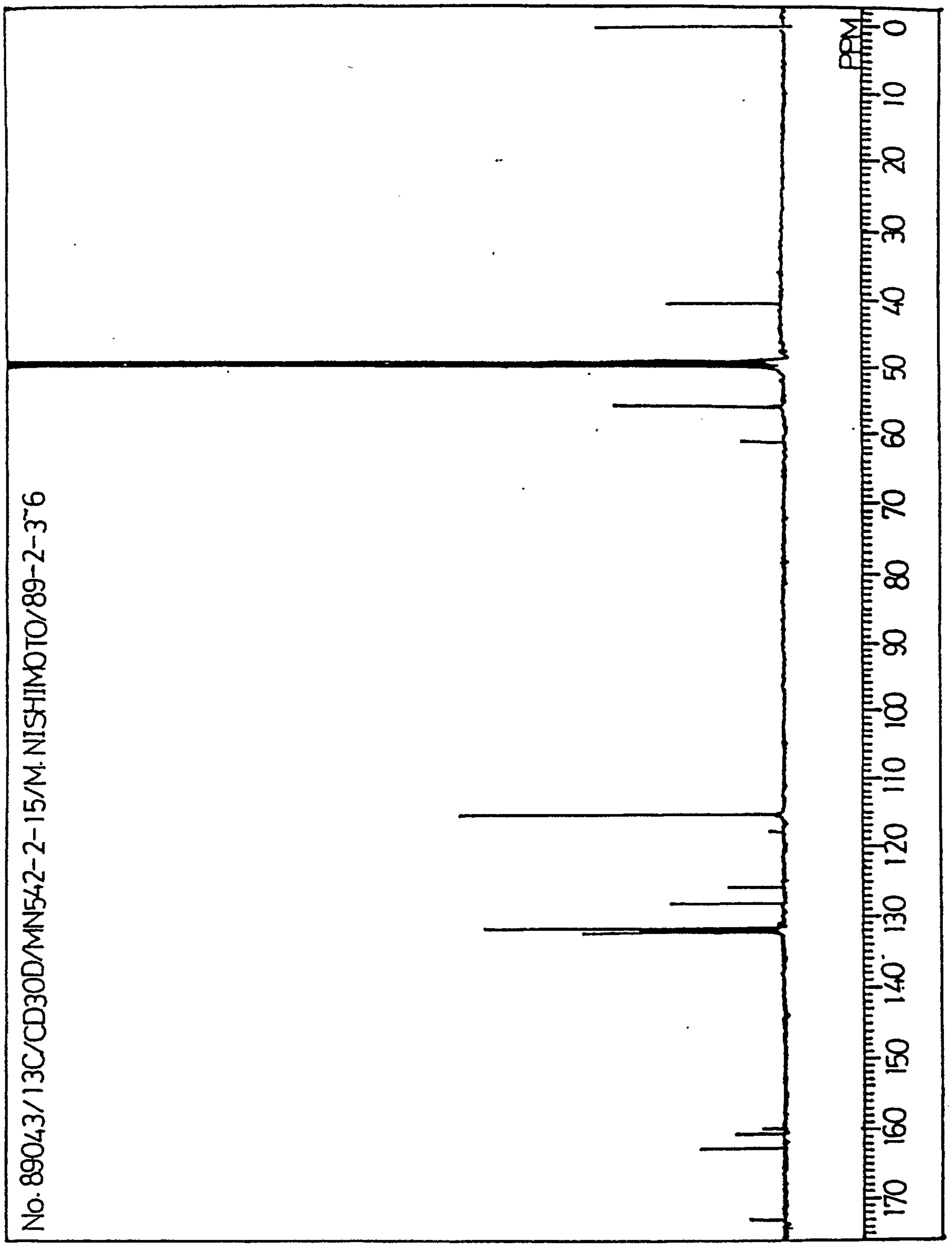
No. 89043/13C/CD3OD/MN542-2-15/M.NISHIMOTO/89-2-3~6
PPM
0
10
20
30
40
50
60
70
80
90
100
110
120
130
140
150
160
170

Fig. 4

Fig. 5

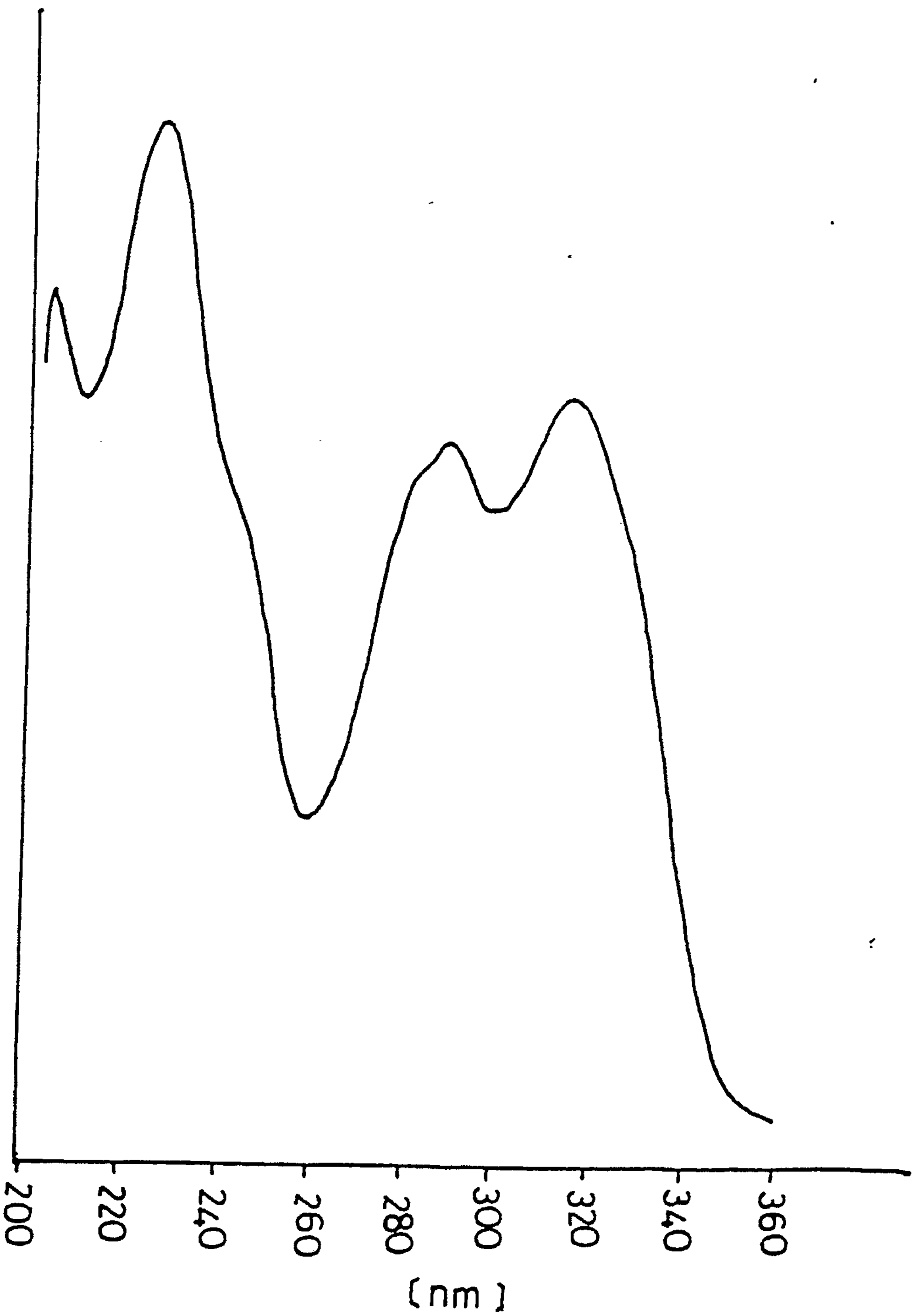

Fig.6

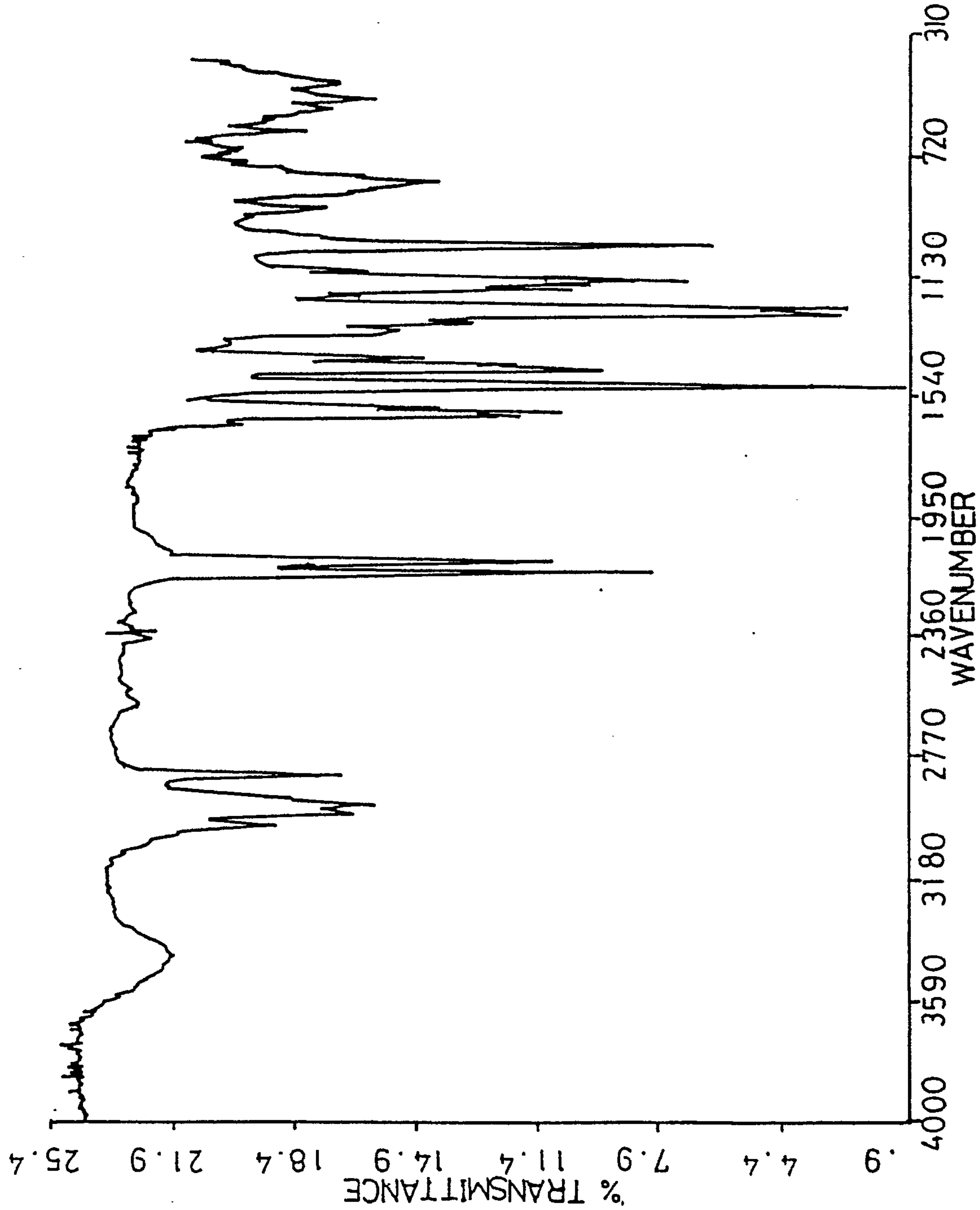
25.4
21.9
18.4
14.9
11.4
7.9
4.4
.9
% TRANSMITTANCE
4000
3590
3180
2770
2360
1950
1540
1130
720
310
WAVENUMBER

Fig. 7

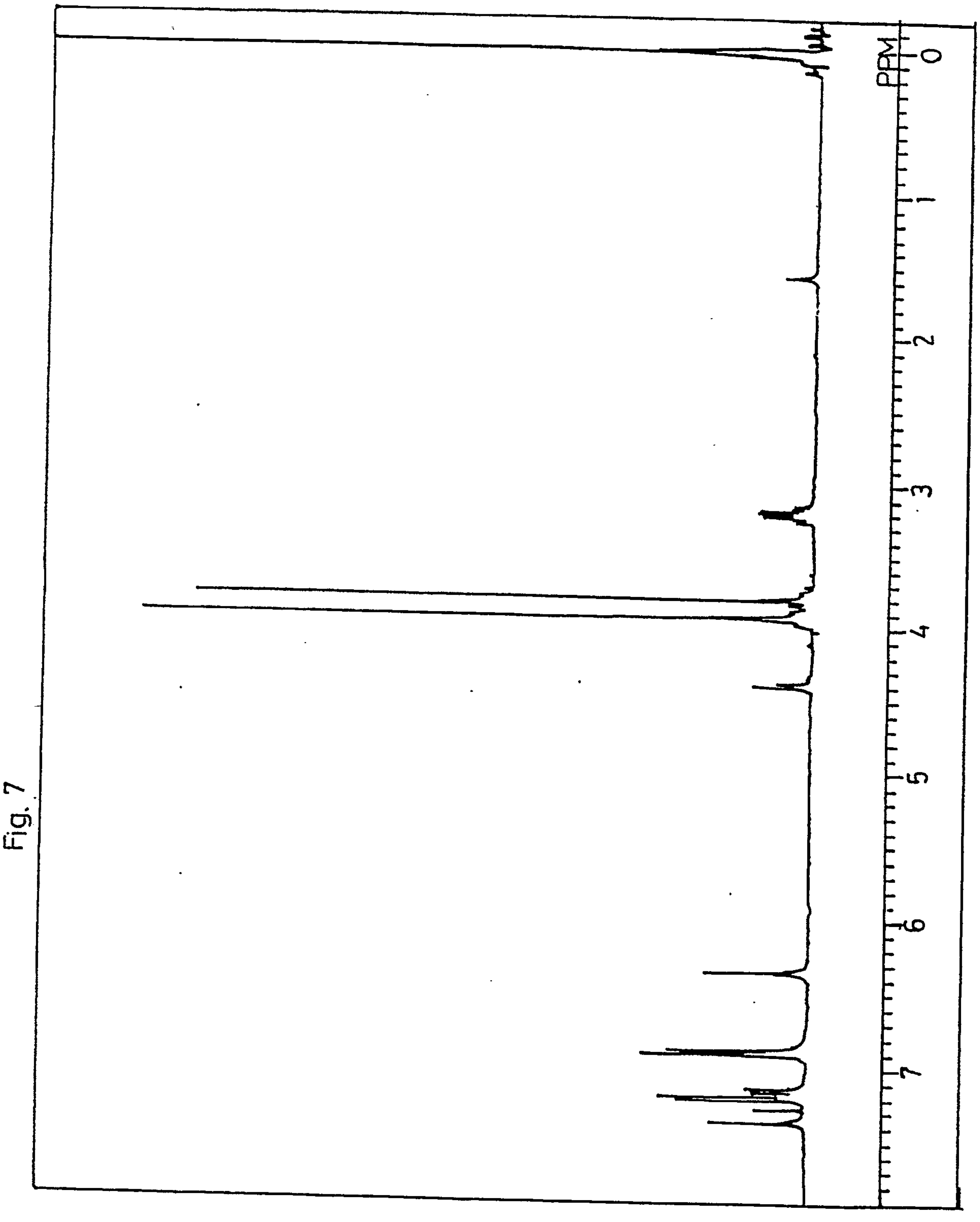

Fig.8

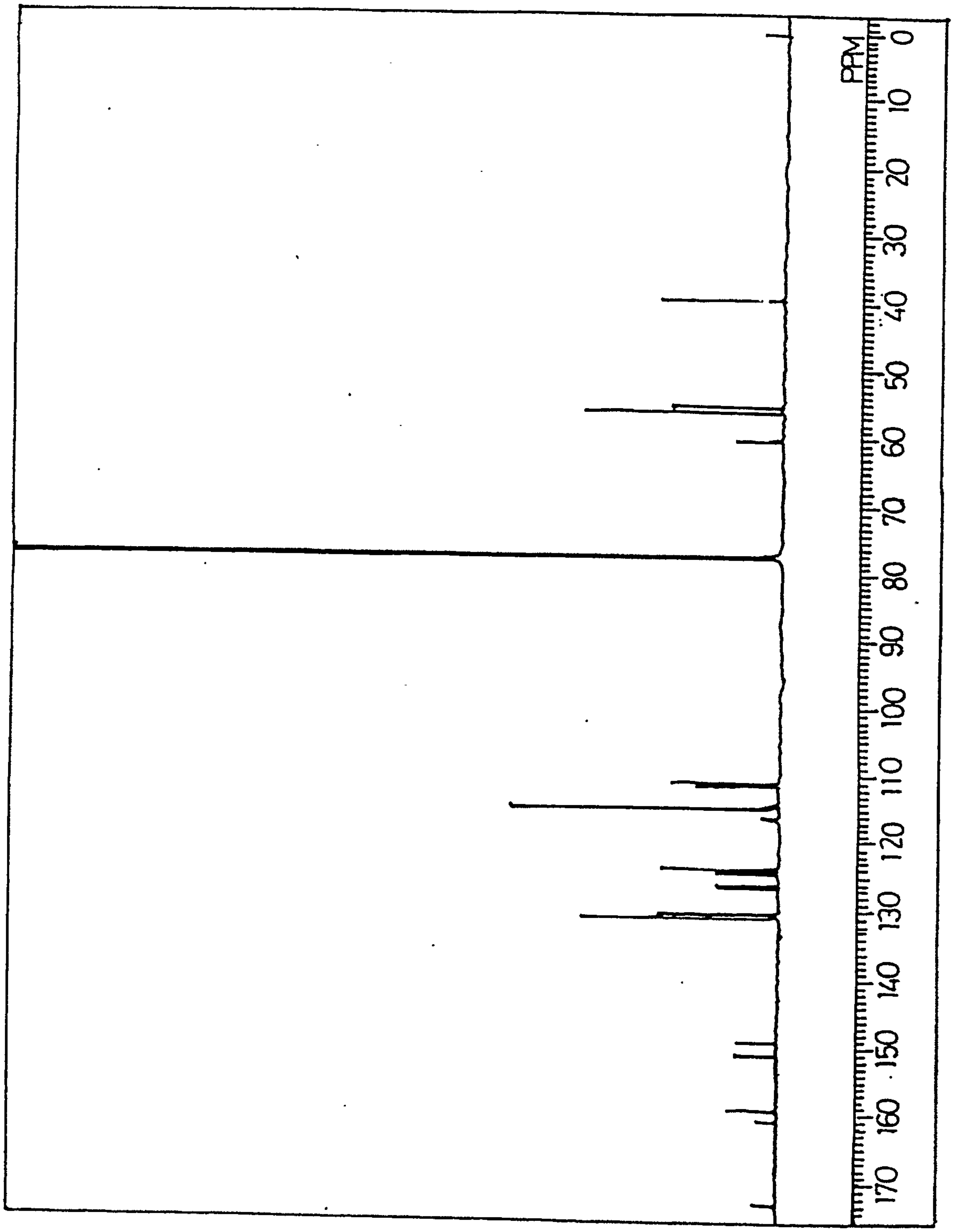

Fig. 9

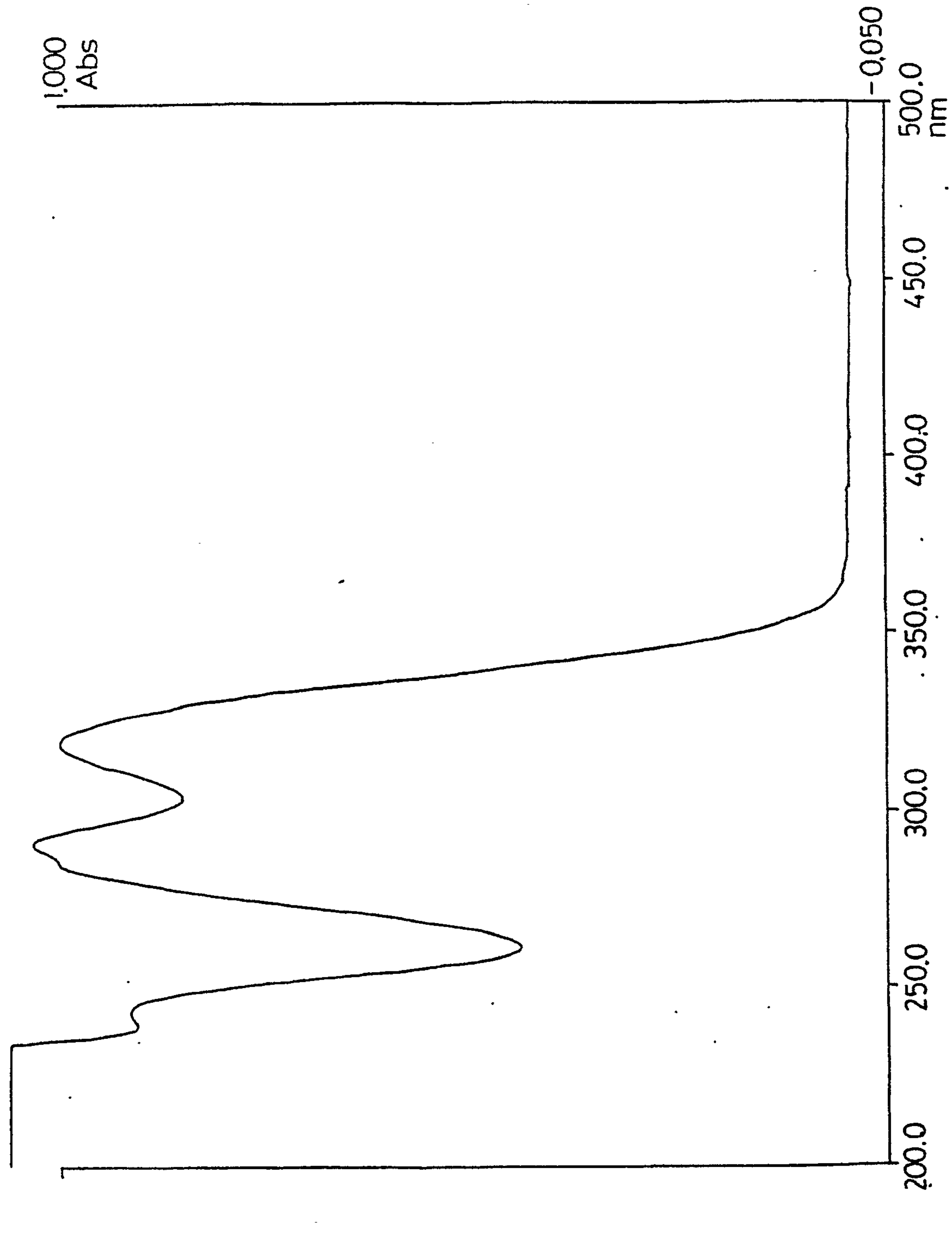

Fig.10

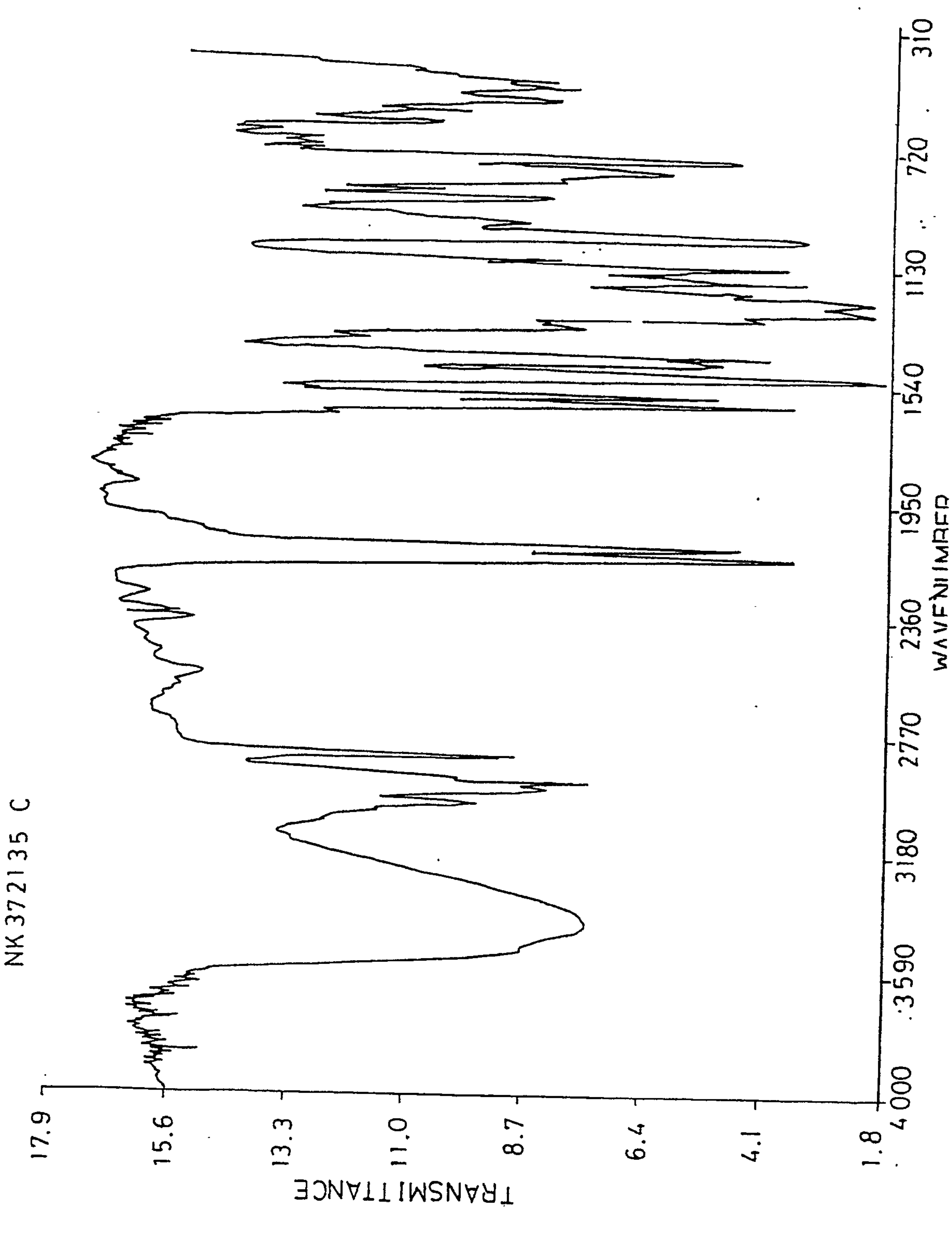
NK 372135 C
TRANSMITTANCE
17.9
15.6
13.3
11.0
8.7
6.4
4.1
1.8
WAVENUMBER
4000
3590
3180
2770
2360
1950
1540
1130
720
310

Fig.11

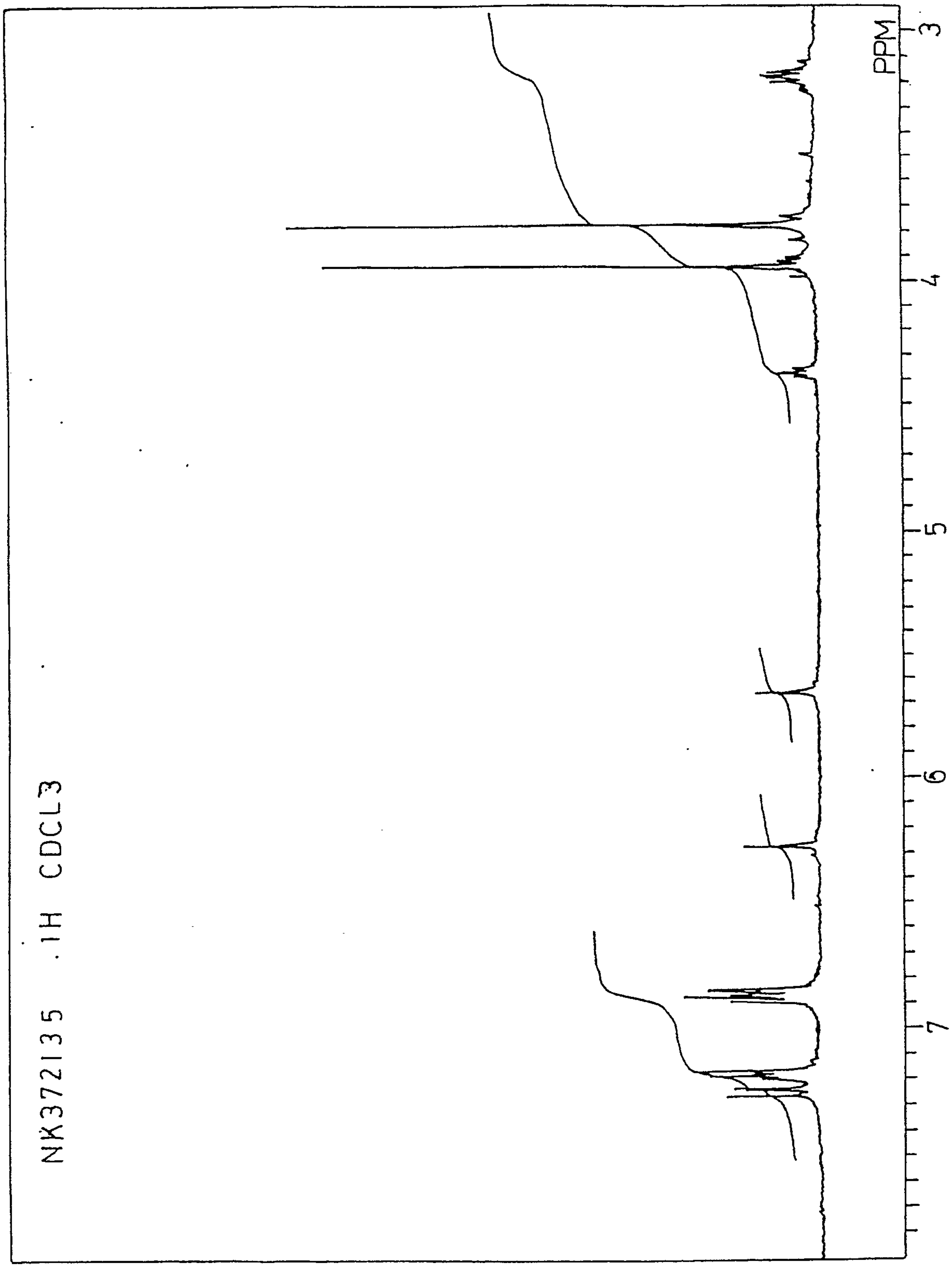

Fig.12

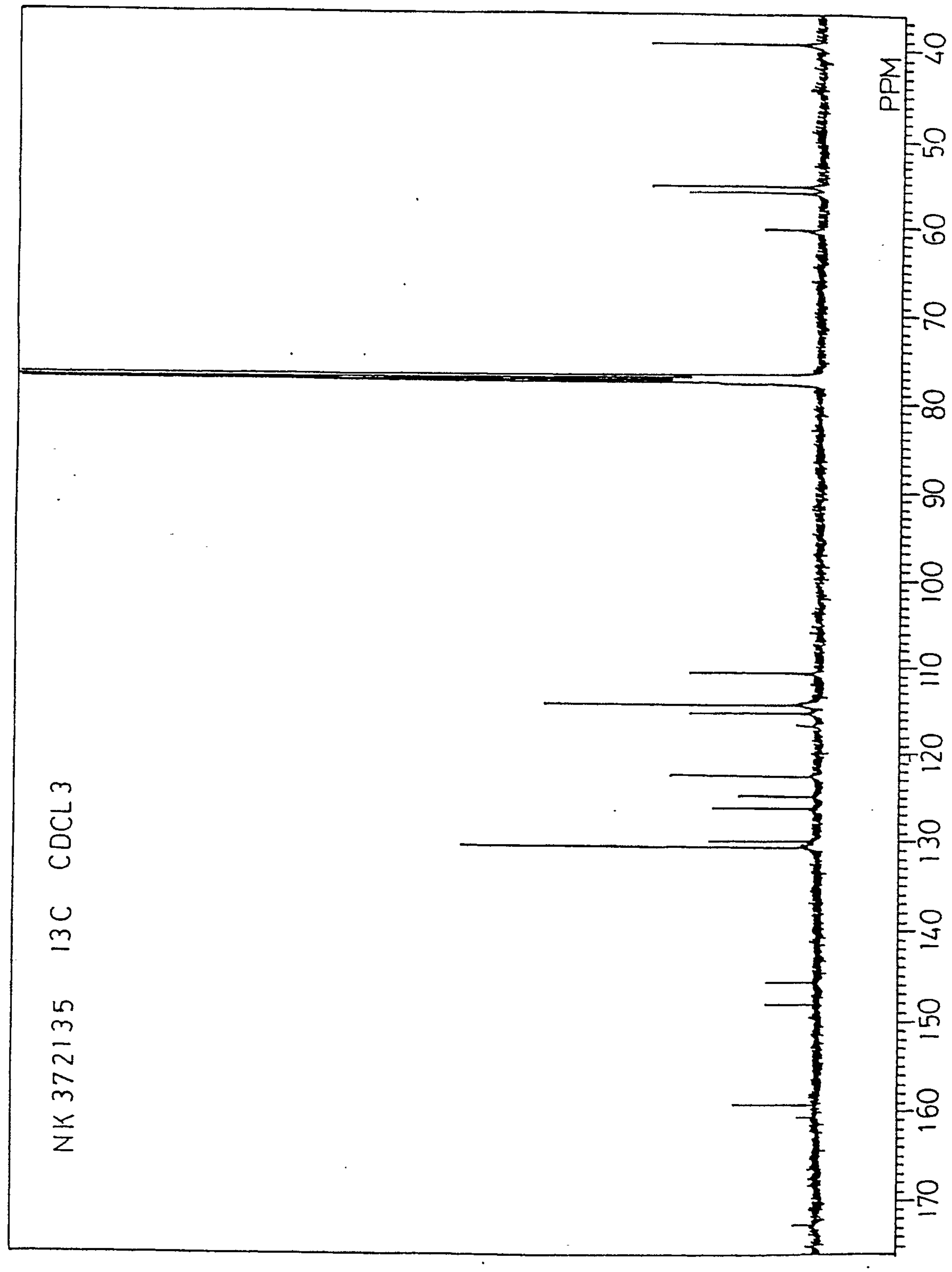
NK 372135 13C CDCL3
PPM
40
50
60
70
80
90
100
110
120
130
140
150
160
170

Fig.13

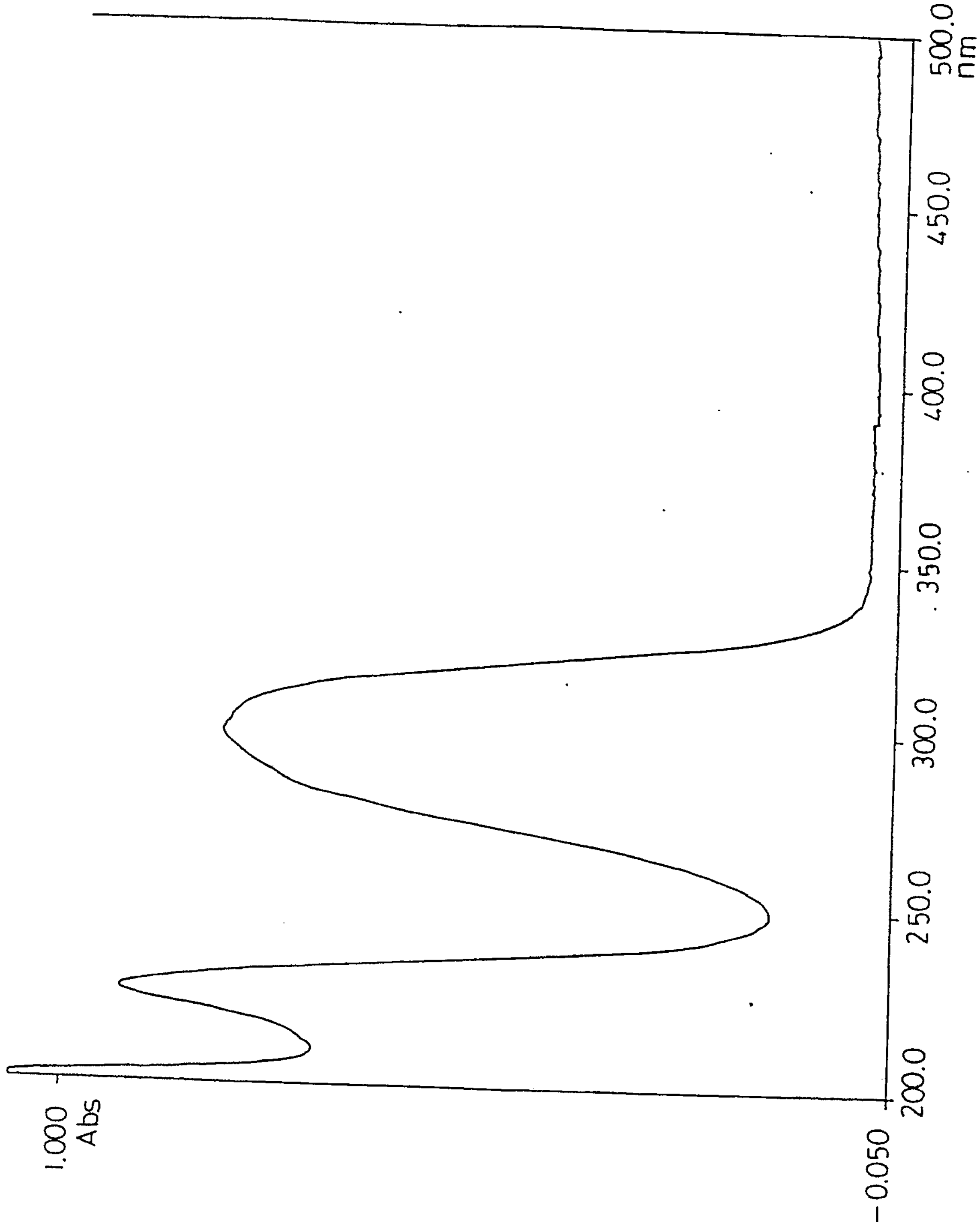

FIG. 14

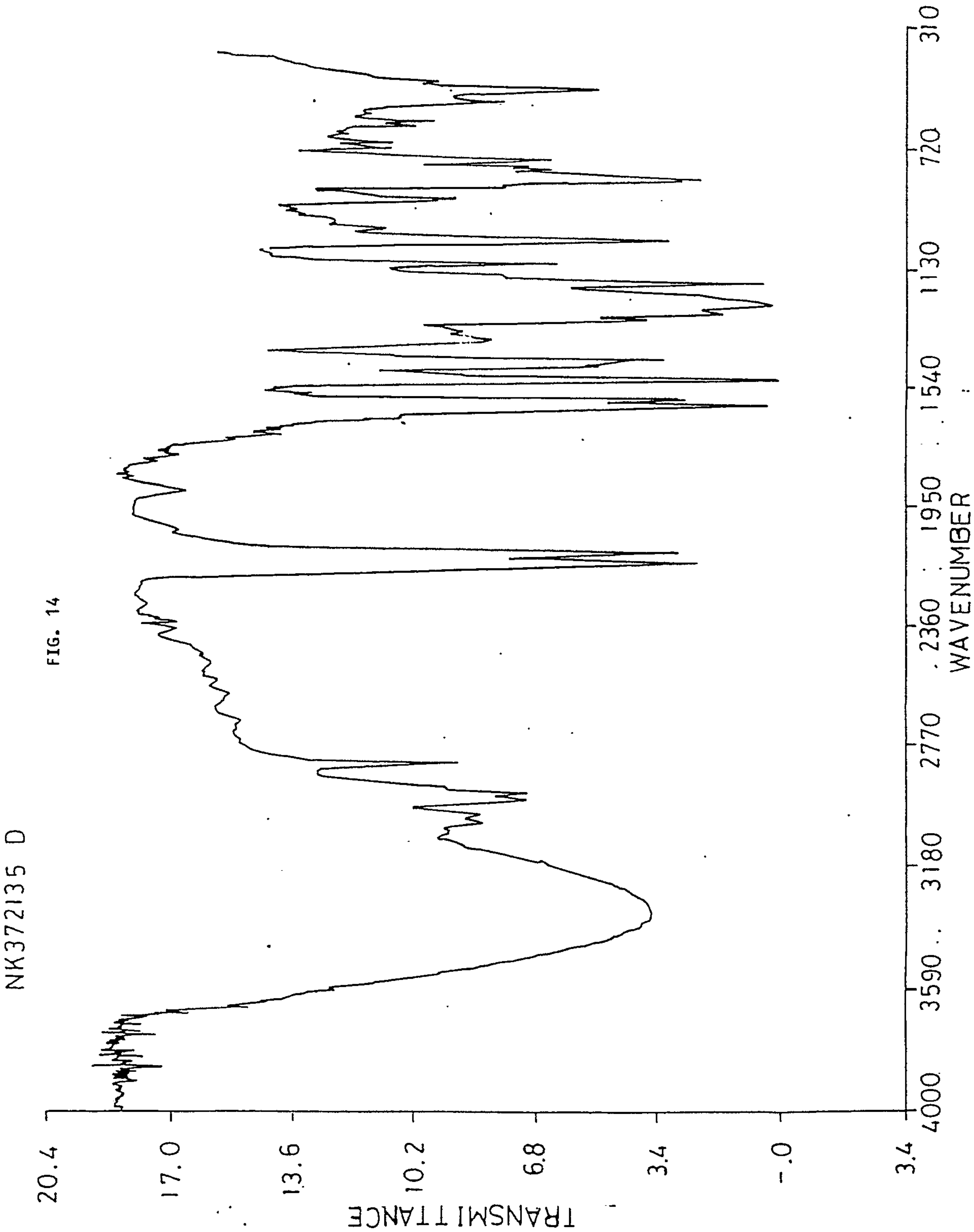
NK372135 D
TRANSMITTANCE
20.4
17.0
13.6
10.2
6.8
3.4
-.0
3.4
WAVENUMBER
4000
3590
3180
2770
2360
1950
1540
1130
720
310

Fig. 15

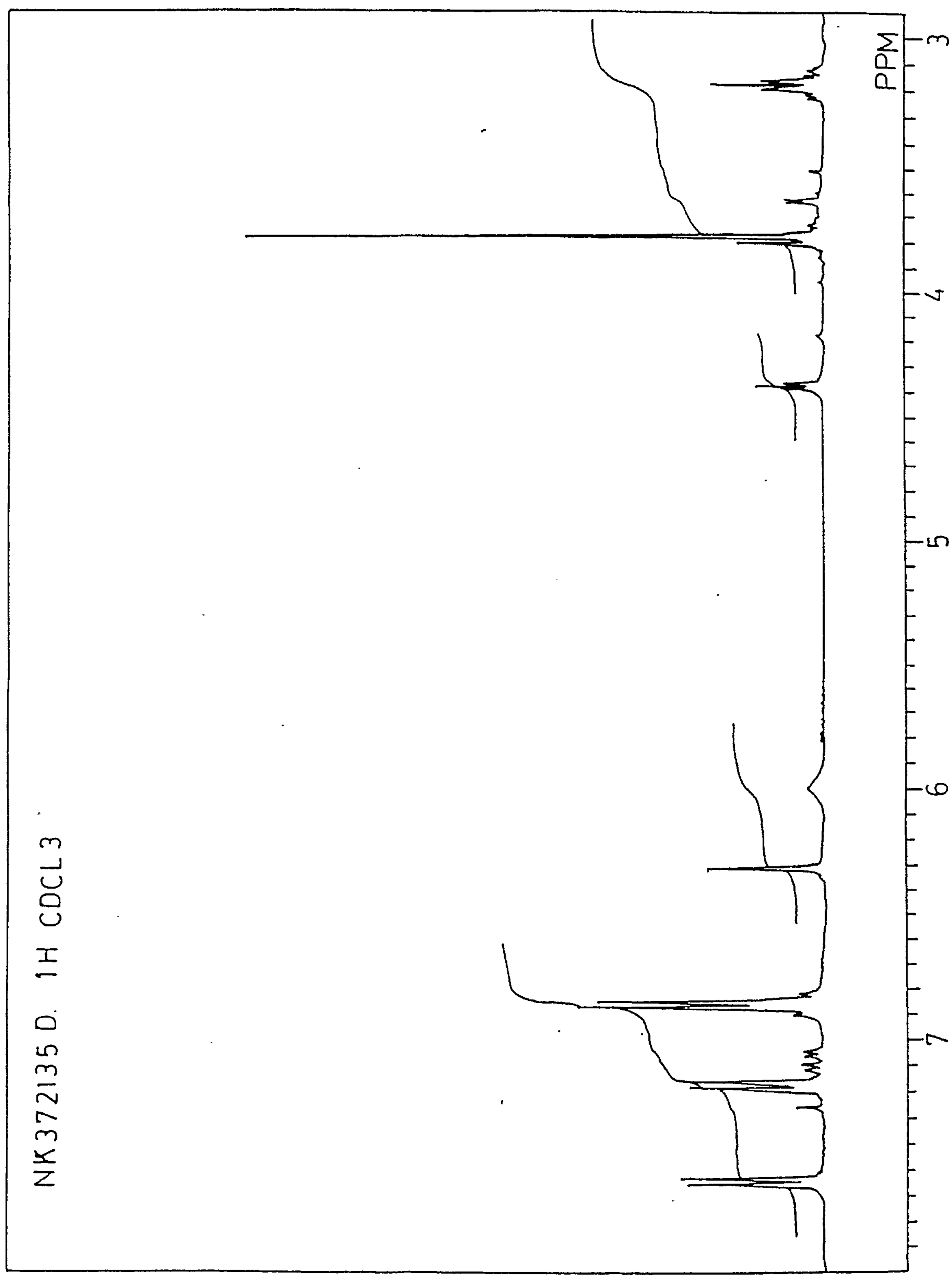

Fig.16

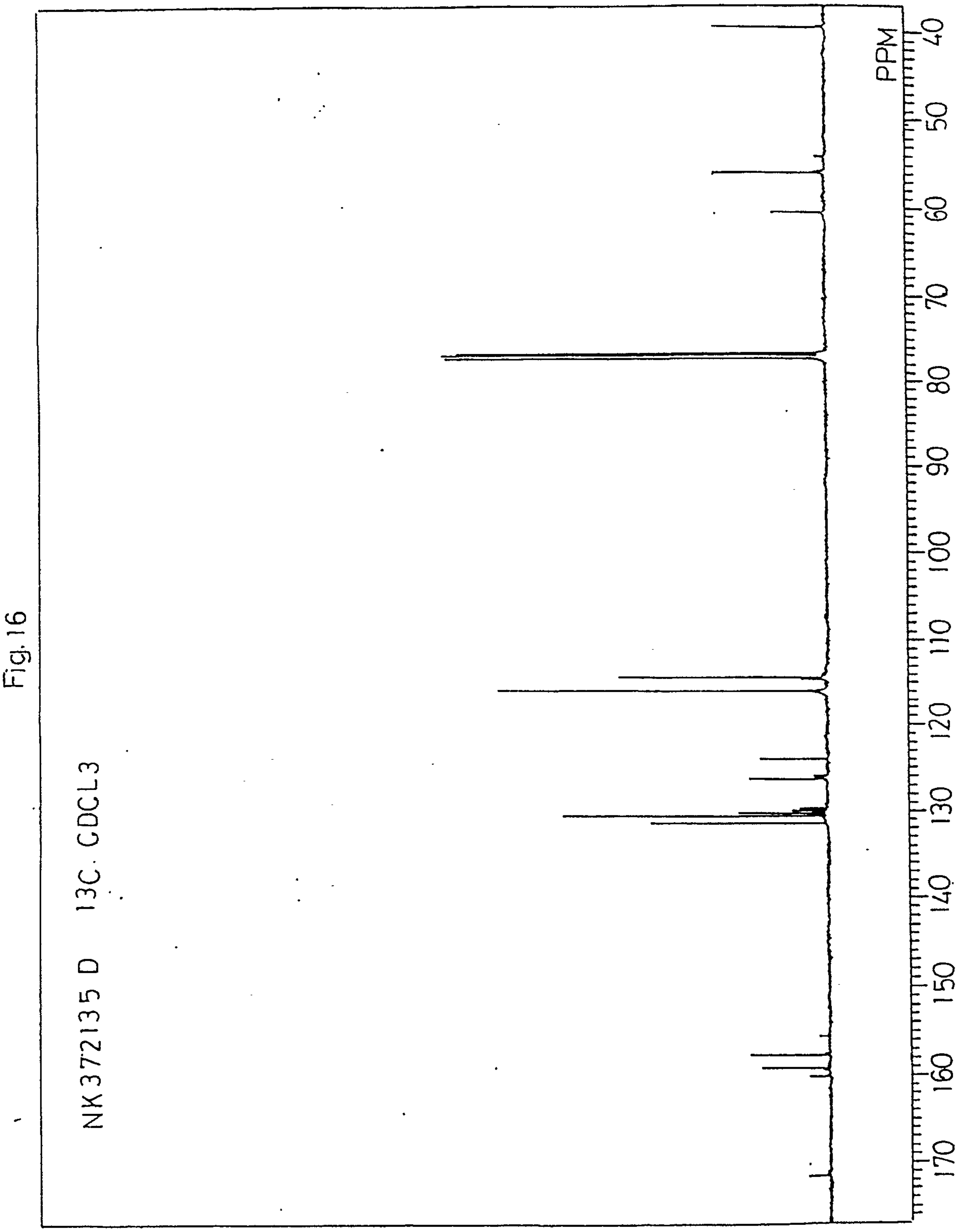
NK372135 D
13C CDCL3
PPM
40
50
60
70
80
90
100
110
120
130
140
150
160
170

European Patent Office

# EUROPEAN SEARCH REPORT

Application Number

EP 90 10 7530

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | CHEMICAL ABSTRACTS, vol. 70, no. 13, 31st march 1969, page 222, abstract no. 56257b, Columbus, Ohio, US; A. TAKATSUKI et al.: "Antiviral and antitumor antibiotics. V. Antiviral antibiotics; xanthocillin X mono- and dimethyl ether, and methoxyxanthocillin X dimethyl ether. 1. Isolation and characterization", & J. ANTIBIOT. (TOKYO) 1968, 21(12), 671-5 --- | 1-6 | C 07 C 291/10<br>A 61 K 31/275<br>A 01 N 47/40<br>C 12 P 13/00 |
| D,A | THE JOURNAL OF ANTIBIOTICS, vol. 34, no. 12, December 1981, pages 1556-1561; K. NOBUAKI et al.: "Xanthocillin X monomethyl ether, a potent inhibitor of prostaglandin biosynthesis" * Page 1556 * ----- | 1-6 | TECHNICAL FIELDS SEARCHED (Int. Cl.5)<br>C 07 C 291/00<br>A 61 K 31/00 |

The present search report has been drawn up for all claims

| Place of search | Date of completion of the search | Examiner |
|---|---|---|
| THE HAGUE | 17-07-1990 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)